# EUROPEAN PATENT APPLICATION

(11) **EP 1 178 119 A2**
(43) Date of publication of application: **06.02.2002**
(21) Application number: 01306444.9
(22) Date of filing: 27.07.2001
(51) Int. Cl.: C12Q 1/68

(54) **A Pcr-based multiplex assay for determining haplotype**

(30) Priority: 31.07.2000 US 221756 P
(71) Applicant: Pfizer Products Inc., Groton, Connecticut 06340 (US)
(72) Inventor: Affourtit, Jason Patrick, Waterford, Connecticut 06385 (US); Seymour, Albert Barnes, Groton, Connecticut 06340 (US)
(74) Representative: Hayles, James Richard

(57) **Abstract**

The invention provides a method for determining haplotype in a template DNA sequence having a first and a second polymorphic marker comprising combining in a single tube a template DNA sequence, forward primers that are allele-specific for the first polymorphic marker, reverse primers that are allele-specific for a second polymorphic marker, and a Stoffel fragment DNA polymerase; conducting polymerase chain reaction (PCR) amplifications to produce an amplification product; and analyzing the amplification product to identify which pair of said forward and reverse primers generated said amplification product; wherein the haplotype is determined by the identification of the forward and reverse primer pair.

SEQUENCE LISTING

<110> Pfizer Products Inc.

<120> A PCR-BASED MULTIPLEX ASSAY FOR DETERMINING HAPLOTYPE

<130> PC10704DAM

<140> 60/221,756

<141> 2000-07-31

<160> 4

<170> PatentIn version 3.0

<210> 1

<211> 25

<212> DNA

<213> Homo sapiens

<400> 1
aggcaatagg ttttgagggg catgg
25

<210> 2

<211> 25

<212> DNA

<213> Homo sapiens

<400> 2
aggcaatagg ttttgagggg catga
25

<210> 3

<211> 21

<212> DNA

<213> Homo sapiens

<400> 3
actccccatc ctccctgctc c
21

<210> 4

<211> 26

<212> DNA

<213> Homo sapiens

<400> 4
gcctgactcc ccatcctccc tgctct
26

## Description

### Field of the Invention

This invention provides methods of using a multiplexed version of an allele-specific polymerase chain reaction (AS-PCR) amplification protocol to determine haplotypes in a single tube assay format.

### Background of the Invention

A haplotype is the *cis* arrangement of alleles for two or more polymorphic markers that are located on a single chromosome. This information is useful, for example, to determine whether a particular haplotype in a population is associated with susceptibility to a disease or condition, or is associated with a variation in response to a drug therapy that is exhibited by a particular sub-population. The analysis of haplotype is also useful to understand the inheritance of a genomic segment within a family or population.

One of the earliest techniques available for determining haplotypes involved studying the inheritance pattern of mapped polymorphic markers within multiple generations of a family tree. More recently, molecular haplotype determination has been made by the identification of polymorphisms at restriction enzyme digestion sites (otherwise known as restriction length polymorphisms or RFLPs) (Bottstein et al., Am. J. Hum. Genet. 32: 314-331, 1980) or by genomic sequencing (Clark, Mol. Biol. Evol. 7: 111-122, 1990). With the advent of polymerase chain reaction (PCR) technology, methods of molecular haplotyping have been developed which directly analyze a single molecule of DNA (Li et al., Nature 335: 414-17, 1988; Ruano et al., Proc. Natl. Acad. Sci. USA 87: 6296-6300, 1990; and Jeffreys et al., Cell 60: 473-85, 1990). However, these PCR-based techniques for haplotyping require multiple PCR reactions using varied combinations of allele-specific primers (Lo et al., Nucleic Acids Res. 19: 3561-67, 1991; Michalatos-Beloin et al., Nucleic Acids Res. 24: 4841-42, 1996; and Sarkar et al., Biotechniques 10: 436-440, 1991).

Despite the number of methods currently available for haplotype determination, none is versatile enough to adapt to a high-throughput format to determine haplotype in virtually any desired template DNA. The present invention overcomes these limitations by providing a method of haplotype determination in a single tube assay format that is suitable for high-throughput screening and can be employed for a wide variety of template DNAs.

### Summary of the Invention

The invention features methods for determining haplotype based upon a multiplex version of AS-PCR that is conducted in a single reaction tube. The methods for determining haplotype in a template DNA sequence containing a first and a second polymorphic marker involve the following: 1) combining in a single reaction tube the template DNA sequence, forward primers that are allele-specific for a first polymorphic marker, reverse primers that are allele-specific for a second polymorphic marker, and a Stoffel fragment DNA polymerase; 2) conducting polymerase chain reaction (PCR) amplifications in the tube to produce an amplification product; and 3) analyzing the amplification product to identify which pair of the forward and reverse primers generated the amplification product. The identification of the primer pair determines haplotype.

Preferably, the method features forward primers that are all fluorescently labelled and reverse primers that have divergent 5' extensions wherein the amplification product analysis comprises gel electrophoresis and fragment analysis. An equally preferred embodiment features forward primers that have divergent 5' extensions and reverse primers that are all fluorescently labelled wherein the amplification product analysis comprises gel electrophoresis and fragment analysis.

In one embodiment of the present invention, the template DNA contains an additional polymorphic marker between the first and second markers. The allelic identity of this additional marker can be included in the determination of haplotype by, for example, restriction fragment length polymorphism (RFLP) analysis or fluorescent depolarization analysis of the amplification product.

By "haplotype" is meant the *cis* arrangement of alleles for two or more polymorphic markers on a particular chromosome, e.g., in a particular gene. The haplotype preserves the information of the phase of the polymorphic markers, i.e., which set of alleles is inherited from one parent and which set from the other.

By "template DNA" is meant a double stranded genomic or cDNA sequence, preferably a human sequence, that is purified to a level sufficient for PCR amplification. A preferred template DNA has at least two markers that are known or expected to be polymorphic in a population, and, most preferably, the polymorphic markers exist in a gene of therapeutic interest.

By a "polymorphic marker" is meant any genomic sequence that varies within a population. This variation includes a single nucleotide polymorphism. An "allele" is one of the variations in the polymorphic marker.

By a "Stoffel fragment DNA polymerase" is meant a modified form of the *Thermus aquaticus* DNA polymerase in which the N-terminal 289 amino acids has been deleted, as further described in Lawyer et al., PCR Methods and Applications, 2: 275-287, 1993, and Tada et al., Cancer Res. 53: 2472-74, 1993 (available as AmpliTaq® DNA polymerase, Stoffel fragment, PE Biosystems, Foster City, CA). The N-terminal deletion eliminates the 5' to 3' exonuclease domain to the DNA polymerase.

By a "reaction tube" is meant a tube, chamber, well, or any other type of vessel in which a PCR reaction is conducted.

By a primer that is "allele-specific" is meant a primer sequence that preferentially hybridizes to one allelic variation of a polymorphic marker. The 3' terminal end of the primer is perfectly complementary to, and thus specific for, the allelic variation.

By a "haplotype associated with the pathogenesis of a disease or condition" is meant a haplotype that is associated with the onset and/or the severity of a symptom of the disease or condition, or is associated with the onset and/or increased rate of progression of the disease or condition. A particular haplotype is associated with a disease or condition when the haplotype segregates with a sub-population suffering from the disease or condition more frequently than do the other haplotypes that exist in the test population as a whole.

By a "disease" or "condition" is meant a state in an individual commonly recognized as abnormal using any objective evidence, such as changes that are evident by physical examination, or the results of diagnostic tests, or by subjective evidence based upon the individual's perception. An abnormality includes a change from normal with regard to function, sensation, or appearance. Included within the terms disease and condition are those listed in standard texts, such as the following: Harrison's Principles of Internal Medicine, 14^{th} Ed., Eds. Fauci et al., McGraw Hill, Columbus, OH, 1997; Robbins Pathologic Basis of Disease, 6^{th} Ed., Eds. Cotran et al., W.B. Saunders & Co., Philadelphia, PA, 1998; and Diagnostic and Statistical Manual of Mental Disorders: Dsm-IV, 4^{th} Ed., American Psychiatric Press, Washington, D.C., 1994.

By "haplotype associated with a varied response to therapy" is meant a haplotype that is involved a phenotypic response to therapy, including the targeted therapeutic response or an inadvertent secondary response, that occurs only in a sub-population of individuals. Such a response may be beneficial or adverse to the patient receiving therapy. A particular haplotype is associated with the response when the haplotype segregates with the sub-population more frequently that do other haplotypes that exist within the total population.

By "therapy" is meant a medical intervention which is intended to produce a beneficial change in a disease or condition in a mammal, preferably a human. A beneficial change includes any one or more of the following: restoration of normal function, reduction of a symptom or an abnormal function, or a slowing in the rate of progression of the disease or condition. Therapy includes administration of a drug, nutritional or behavioral modification, or the administration of radiation, or a combination of more than one of any single therapy.

By a "drug" is meant a chemical entity or biological product, or a combination of chemical entities and/or biological products, administered to a person to treat, prevent, retard, or reverse the progression of a disease or condition. Included within the term "drug" are any chemical compounds, such as, for example, low molecular weight compounds, nucleic acids, oligonucleotides, amino acids, polypeptides, glycoproteins, lipoproteins, ribozymes, DNAzymes, and monoclonal or polyclonal antibodies, or fragments thereof, such as variable chain fragments.

The methods of this invention provide for a number of advantages related to their simplicity and versatility in determining haplotype. For example, given that the methods can be performed in a single tube assay, the methods are readily adapted to a high-throughput format. This feature is a significant improvement over previously reported PCR-based methods of haplotyping which require several separate PCR reactions with different primer pair combinations in order to elucidate the haplotype (e.g., Lo et al., Nucleic Acids Res. 19: 3561-67, 1991; Michalatos-Beloin et al., Nucleic Acids Res. 24: 4841-42, 1996; and Sarkar et al., Biotechniques 10: 436-440, 1991). In addition, the present methods have advantages over population-based (e.g., Jorde, Am. J. Hum. Genet. 56: 11-14, 1995; Thomson, Am. J. Hum. Genet. 57: 474-86, 1995; de la Ghapelle, J. Med. Genet. 30: 857-65, 1993; Weber and May, Am. J. Hum. Genet. 44: 388-96, 1989) and RFLP-based (Bottstein et al., Am. J. Hum. Genet. 32: 314-331, 1980) methods of haplotyping because the present methods can be used to haplotype a broader range of template DNAs. For example, haplotype determinations can be made in template DNA regardless of whether the polymorphic markers are in strong linkage disequilibrium (in contrast to population-based haplotyping) and regardless of the existence of restriction enzyme recognition sites between the polymorphic markers of interest (unlike RFLP-based haplotyping).

Other features and advantages of the invention will be apparent from the following detailed description and from the claims. All publications herein are incorporated by reference in their entireties.

### Description of the Figures

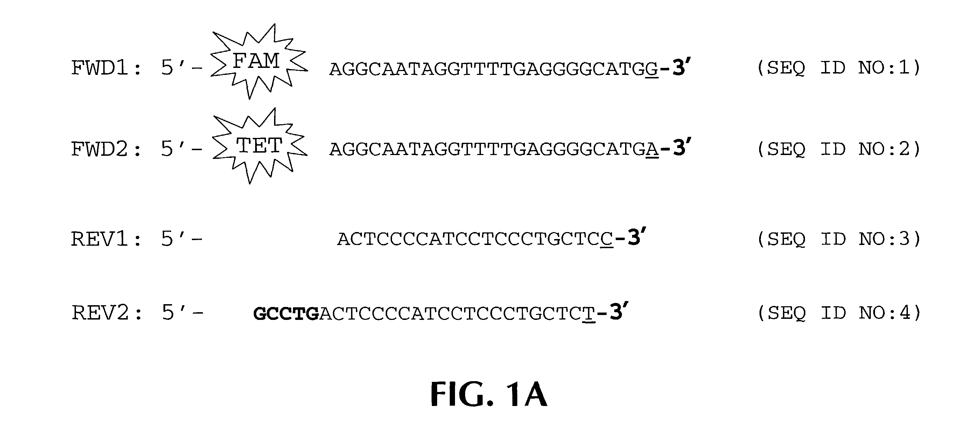

Figure 1A shows the sequences of the two forward primers (FWD1 and FWD2) and the two reverse primers (REV1 and REV2) used for haplotyping the promoter region of the human tumor necrosis factor-α (TNFα) at the -308 and -238 positions. FWD1 and FWD2 are labelled with carboxyfluorescein (FAM) and tetrachlorofluorescein (TET), respectively. The 5' extension of REV2 with respect to REV1 is highlighted in bold.

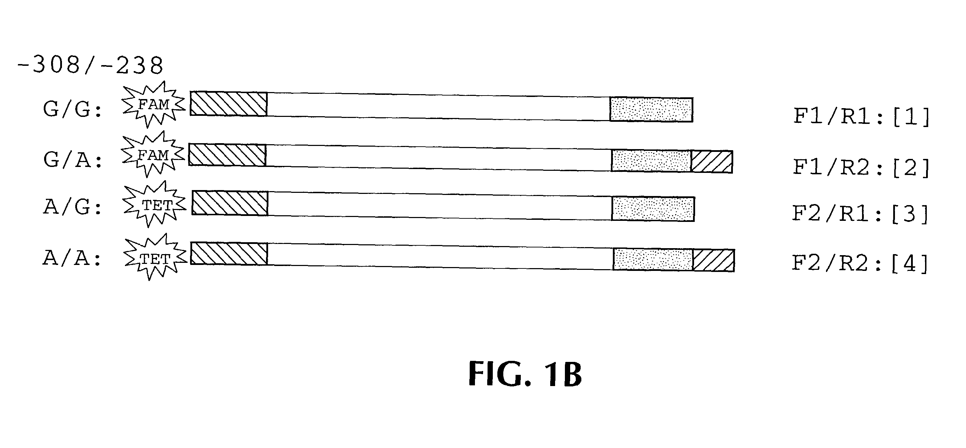

Figure 1 B is a schematic demonstrating the distinguishable characteristics of amplification products produced by the four possible pairings of the forward and reverse primers in terms of fluorescent label and fragment length.

### Detailed Description

The present invention features methods for determining a haplotype of interest in a mammal, preferably a human, using a multiplex version of AS-PCR. In contrast to other AS-PCR methods of haplotyping, the template DNA and all allele-specific primers are combined in a single reaction tube for a multiplex PCR assay; the amplification product generated is then analyzed to call the haplotype of the template DNA.

The template DNA is double-stranded DNA containing at least a first and second polymorphic marker from the same chromosome. The PCR primers comprise sets of forward and reverse primers that are allele-specific for the first and second marker, respectively. The allelic specificity of the primers within each set is created by diverging nucleotides at the 3' termini of the primers. This divergence renders each primer uniquely complementary to one of the allelic variations of the polymorphic marker (see, e.g., Ugozzoli and Wallace, Genomics 12: 670-74, 1992). A complete set of primers includes one primer to match to each allelic variation of the polymorphic marker.

The PCR amplification is conducted under conditions which allow any theoretically possible primer pair to amplify the template DNA, provided that the primer extension only occurs with high specificity (i.e., inducing PCR amplification only when the 3' ends of the primers are perfectly matched to the template DNA). These conditions are essential for accurate haplotyping by the present methods because the identification of the forward and reverse primer pair used in amplification reveals the haplotype at the first and second polymorphic marker (i.e., PCR amplification with mismatched primers will lead to improper determination of the alleles present in the template DNA).

A key feature of the present invention that provides the desired high specificity PCR amplification without compromising the efficiency of any possible primer pair extension is the Stoffel fragment DNA polymerase ("Stoffel fragment"). The Stoffel fragment is a truncated form of *Thermus aquaticus* DNA polymerase that lacks the N-terminal 289 amino acids and is devoid of 5'-3' exonuclease activity (Lawyer et al., PCR Methods and Applications 2: 275-87, 1993, Tada et al., Cancer Res. 53: 2472-74, 1993; commercially available as AmpliTaq® DNA polymerase, Stoffel fragment, PE Biosystems, Foster City, CA).

Amplification reactions using the Stoffel fragment are carried out using standard PCR techniques (see, e.g., Arnheim and Erlich, Annu. Rev. Biochem. 61: 131-56, 1992; Ausubel et al., Current Protocols in Molecular Biology, Wiley and Sons, New York, NY, 1999). For example, PCR reactions are carried out in a small total volume (e.g., 25-100 µl) containing the Stoffel fragment (at approximately 0.5-1 units/µl), template DNA (at approximately 0.25-10 ng/µl), each of the four deoxynucleoside triphosphates (dNTPs, i.e., dATP, dCTP, dGTP, and dTTP, at approximately 50-200 nM each), each of the forward and reverse allele-specific DNA primers (at approximately 50-500 nM each), suitable salts and buffers (e.g., 10-60 mM KCI, 1.5-4 mM MgCl₂, 10 mM Tris, pH 8.3-8.4), and, preferably, dimethylsulfoxide (DMSO) (at approximately 1-5%). To ensure that all appropriate allele-specific PCR reactions are detectable within the multiplexed assay, those skilled in the art will understand, based on the present description, how to modifiy the concentration of the primers to compensate if a bias is detected between primers in terms of preferential amplification or label intensity. Thus, concentrations of the primers may vary by 5-15 fold.

PCR is performed in a PCR thermal cycler (e.g., MJ Research, Waltham, MA; Perkin Elmer, Norwalk, CT). Cycling parameters include an initial denaturation step (e.g., 95°C for 3-5 minutes) followed by multiple cycles (e.g., 25-40 cycles) of denaturation at 95°C for one minute, annealing for 30 seconds at 50-68°C (depending on the annealing temperature of the primers), and extension at 72°C for 1 minute. The PCR amplification concludes with a final extension step (e.g., 72°C for 5-10 minutes).

The template DNA used for the present invention is PCR-quality genomic DNA that is isolated, for example, by alkaline-lysis DNA preparation (e.g., Current Protocols in Human Genetics, John Wiley and Sons, New York, NY, 1999; Engelstein et al., Microbial and Comparative Genomics 3: 237-41, 1998; Wirgart et al., Clinical and Diagnostic Virology 7: 99-110, 1996). DNA isolation kits are commercially available (e.g., Puregene® DNA isolation kit, Gentra Systems, Inc., Minneapolis, MN).

The customized primers used for PCR amplification can be obtained commercially (PE Biosystems, Foster City, CA) or can be synthesized using standard phosphoramidite methods known in the art (e.g., Current Protocols in Molecular Biology, John Wiley & Sons, New York, NY, 1999). Preferred primers are 14-30 nucleotides, more preferably, 16-24 nucleotides in length. In general, all primers contemplated for use in the single tube PCR reaction should be designed to have similar annealing temperatures.

In the present methods, the identification of which primer pair in the reaction tube generated the PCR amplification product is possible by designing a unique characteristic into each forward and reverse primer such that every possible primer pairing confers a unique combination of characteristics on the resultant PCR amplification product. Examples of schemes to uniquely tag each primer in a set of primers include tagging the primers with distinguishable radiolabels, distinguishable fluorescent labels, and varying the length of the primers at their 5' end. It is preferred to tag one set of forward or reverse primers with a fluorescent label and the other set by divergent 5' extension.

Examples of suitable fluorescent labels include 5-carboxyfluorescein (5-FAM), 6-carboxyfluorescein (6-FAM), carboxyrhodamine 110 (R110), carboxyrhodamine 6G (R6G), hexachlorofluorescein (HEX), carboxy-X-rhodamine (ROX), N-(1-napthyl)-ethylenediamine dihydrochloride (NED), tetrachlorofluorescein (TET), carboxytetramethylrhodamine (TAMRA), and 6-carboxy-4',5'-dichloro-2',7'-dimethoxyfluorescein (JOE).

With respect to 5' primer extension, the preferred primer extension is non-complementary to the template DNA and is not self-complementary (which prevents the primer from looping back and binding to itself). These primers of different lengths produce PCR amplification products of varying lengths which can be distinguished based upon different electrophoretic mobility profiles. It is preferred that the 5' extensions are no longer than necessary to distinguish the amplification products because the longer the primer extension, the greater the potential for altering PCR efficiency. Typically, primer discrepancies of 5-10 nucleotides are sufficient.

By using the above-described combination of fluorescently-labelled and divergent 5' extended primers to label the forward and reverse sets of primers, it is possible to conduct PCR amplification in a single reaction tube and identify which primer pair was actually used to generate the resultant PCR amplification product following gel electrophoresis and fragment analysis. The PCR products are diluted in water to the desired concentration (e.g., 10-20 fold), mixed with a control DNA ladder for size reference (e.g., TAMRA® GeneScan-350 DNA ladder, PE Biosystems, Foster City, CA), and blue dextran loading dye (Sigma Chemical Co., St. Louis, MO), then heated to 95°C for 5 minutes, and placed on ice. A small sample (e.g., 1-2 µl) from this mixture is loaded onto an acrylamide sequencing (slab or capillary) gel (e.g., a Long-Ranger® 36 cm, 32-96 well, 5% acrylamide gel; BioWhittaker Molecular Applications, Rockland, ME; large fragments >1kB may require a lower % acrylamide gel for adequate separation) and run on an appropriate electrophoresis system coupled to a laser/CCD instrument for detection of fluorescence (e.g., slab-gel electrophoresis instruments include the ABI 377 Prism® Fluorescent Sequencer, capillary-gel electrophoresis instruments include ABI Model 310/343 and ABI Model 3700; all are available from Applied Biosystems, Foster City, CA; comparable systems are available from Licor, Lincoln, NE).

These electrophoretic instruments are tuned for fragment analysis using run parameters, for example, the filter used, voltage, current, power, gel temperature, and laser power, that are optimized for the dyes used and the desired run length, as those skilled in the art will appreciate.

The gel files created by the electrophoresis of the amplification products are analyzed using software such as GeneScan® versions 2.0, 2.1, or 3.0 (PE Biosystems, Foster City, CA). Using this software, data analysis includes removing spectral overlap between the dyes, multicomponent analysis, background subtraction, and fragment size estimation based on a comparison of migration rate to controls of known size. Haplotype can be determined by visual inspection of the GeneScan® results. Alternatively, automated haplotype calling can be performed on the GeneScan® results using software such as GenoTyper® (PE Biosystems, Foster City, CA).

In the event that unexpected amplification products are detected, which indicates that non-specific PCR amplification occurred, the PCR conditions may be optimized to increase specificity according to standard methods (see, e.g., Chou et al., Nucleic Acids Res. 20: 1717-23, 1992; Higuchi et al., BioTechnology 11: 1026-30, 1993; Persing and Cimino, Amplification Product Inactivation Methods, In: *Diagnostic Molecular Microbiology: Principles and Applications,* Eds. Persing et al., American Society for Microbiology, Washington, D.C., 1993, p. 105-21). These methods include increasing the annealing temperature, lowering the salt concentration, and/or adding additional cosolvent.

As would be understood by those skilled in the art based on the present description, the above-described methods of haplotyping can be modified to determine the haplotype at more than two markers in template DNA using RFLP or fluorescent polarization. For example, the allelic identity at a third polymorphic marker that is internal to the first and second marker can be discriminated via restriction enzyme digestion of the amplification products if the polymorphic marker exhibits an allele-dependent restriction enzyme recognition site (see, e.g., Current Protocols in Human Genetics, John Wiley & Sons, New York, NY, 1999). According to this embodiment, amplification products are subjected to digestion by the appropriate restriction enzyme prior to electrophoresis.

The haplotyping methods of the present invention can be used to identify haplotypes in individuals without knowledge of genotype information from ancestral generations. In addition, the methods can identify particular haplotypes that are associated with a disease or condition if the haplotype is significantly overrepresented in a population subgroup that exhibits the disease or condition. Alternatively, the methods can be used to identify haplotypes that may be associated with a variation in drug response that occurs within a sub-population of patients. Such determination is useful, for example, to better evaluate drug efficacy and/or to reduce the occurrence of potential side effects.

In addition, once a haplotype is identified as associated with a disease or condition, using either the methods of the present invention or standard population-based linkage disequilibrium studies, then the haplotyping assay can be used as a diagnostic to determine whether an individual suffers from the disease or condition or is at risk for developing the disease or condition. Such diagnostic methods are intended, most preferably, for humans, but may also be applied in other mammalian species.

### Example: Haplotype determination of the TNFα gene promoter region

Haplotype determinations were made by a method of the present invention for polymorphic markers present in the TNFα gene of sixteen individuals. These results revealed that three of the four possible haplotypes were present in the test population, and matched haplotype determinations of the same individuals using standard RFLP technology with 100% concordance. The TNFα gene was chosen for study because it encodes a proinflammatory cytokine associated with a broad variety of diseases such as asthma, infectious diseases, obesity, and arthritis. In addition, this gene exhibits well known polymorphisms at positions -308 (G or A) and -238 (G or A) of the promoter region.

Template DNA was isolated from Coriell Cell Repository (Camden, NJ) subjects using the Puregene® DNA isolation kit (Gentra Systems, Inc., Minneapolis, MN, Cat. D-5500A).

The AS-PCR technique of the present invention was used to distinguish the four possible haplotypes for the -308 and -238 markers using two fluorescently-labelled forward primers and two reverse primers with divergent 5' extensions (Fig. 1A). The two forward allele-specific primers differentially matched the G or A allele at position -308 (-308G or -308A, respectively). Forward primer 1 (FWD1; SEQ ID NO: 1) was labelled with FAM and matched genes having -308G; forward primer 2 (FWD2; SEQ ID NO: 2) was labelled with TET and matched genes having the -308A allele. The two reverse allele-specific primers differentially matched the G or A allele at position -238. Reverse primer 1 (REV1; SEQ ID NO: 3) matched TNFα genes having the -238G allele; reverse primer 2 (REV2; SEQ ID NO: 4) had a five nucleotide extension at its 5' end with respect to REV1 and matched TNFα genes having the -238A allele.

The custom primers were obtained commercially (PE Biosystems, Foster City, CA). The PCR reactions were carried out in a single tube in a total volume of 25 µl following the optimization of PCR reactions and cycling conditions using standard techniques (see, e.g., Chou et al., Nucleic Acids Res. 20: 1717-23, 1992; Higuchi et al., BioTechnology 11: 1026-30, 1993; Persing and Cimino, Amplification Product Inactivation Methods, In: *Diagnostic Molecular Microbiology: Principles and Applications,* Eds. Persing et al., American Society for Microbiology, Washington, D.C., 1993, p. 105-21). In addition to the template DNA (100 ng), each 25 µl PCR reaction sample contained 1X Stoffel buffer (10 mM KCI, 10 mM Tris-HCI, pH 8.3, PE Biosystems, Foster City, CA), 2 units Stoffel fragment AmpliTaq® polymerase (Lawyer et al., PCR Methods and Applications 2: 275-87, 1993, PE Biosystems, Foster City, CA), 55 nM of each dNTP, additional KCI for a final concentration of 60 mM, 5% DMSO, 0.8 mM MgCl₂, and each of the four above-described allele-specific primers at concentrations of 0.4 mM (FWD1, FWD2, and REV1) and 0.08 mM (REV2). The REV2 primer concentration was reduced as compared to the REV1 primer concentration to enable detection of the weaker signal generated by REV1 amplification products.

The PCR thermal cycling conditions were as follows: initial denaturation occurred at 95°C for five minutes; 30 subsequent cycles of denaturation, annealing, and extension occurred at 95°C for 1 minute, 64°C for 30 seconds, and 72°C for one minute, respectively; and the final cycle of extension occurred at 72°C for 10 minutes.

These PCR products were diluted 10 fold in deionized water. A 5 µl aliquot was taken, mixed with 1 µl TAMRA GeneScan-350 DNA ladder (PE Biosystems, Foster City, CA) and 1 µl blue dextran loading dye (Sigma Chemical Co., St. Louis, MO), heated to 95°C for 5 minutes, and then placed on ice. A 1.5 µl sample from this mixture was loaded onto an acrylamide slab-gel (Long-Ranger® 36 cm, 5.0% acrylamide gel, BioWhittaker Molecular Applications, Rockland, ME, Cat. No. 50691) and run on an ABI Model 377XL Fluorescent Sequencer electrophoresis system coupled to a laser/CCD instrument for detection of fluorescence. The instrument parameters were set at 3000 volts, 60 mAmps, 200 watts, 51°C, a "C" virtual filter, a laser power of 40 mWatts, and a run time of 2 hours (GeneScan-2400 run parameters, Firmware ver. 2.0, Collection ver. 2.0, PE Biosystems).

Gel file analysis was conducted using GeneScan® ver. 3.1 and automated haplotyping was called with Genotyper® software (PE Biosystems)

The haplotype determinations of the polymorphic markers at positions-308 and -238 of the TNFα gene were determined in the individual DNA samples as follows (see also Fig. 1B): (1)TNFα genes with haplotype 1 (G alleles at both the-308 and -238 positions (G/G)) produced amplification products that emitted blue fluorescence (FAM labelled) and exhibited a fragment length of 115 base pairs from amplification by the primer pair FWD1/REV1; (2) TNFα genes with haplotype 2 (a G allele at the -308 position and an A allele at the -238 position (G/A)) produced blue amplification products with a length of 120 base pairs resulting from amplification of the FWD1/REV2 combination of primers; (3) TNFα genes with haplotype 3 (an A allele at the -308 position and a G allele at the -238 position (A/G)) produced green amplification products (TET labelled) of 115 base pairs resulting from amplification of the FWD2/REV1 combination of primers; and (4) TNFα genes with haplotype 4 (A alleles at both the -308 and -238 positions (A/A)) produced green amplification products of 120 base pairs based upon amplification of the FWD2/REV2 combination of primers.

### Other Embodiments

While the invention has been described in connection with specific embodiments, it will be understood that it is capable of further modifications. Therefore, this specification is intended to cover any variations, uses, or adaptations of the invention that follow, in general, the principles of the invention, including departures from the present disclosure that come within known or customary practice within the art.

## Claims

1. A method for determining haplotype in a template DNA sequence comprising a first and a second polymorphic marker, said method comprising:
i) combining in a single reaction tube said template DNA sequence, forward primers that are allele-specific for a first polymorphic marker, reverse primers that are allele-specific for a second polymorphic marker, and a Stoffel fragment DNA polymerase;
ii) conducting polymerase chain reaction (PCR) amplifications in said tube to produce an amplification product; and
iii) analyzing the amplification product to identify which pair of said forward and reverse primers generated said amplification product;
wherein the haplotype is determined by the identification of said forward and reverse primer pair.

2. The method of claim 1, wherein said template DNA comprises an additional polymorphic marker between said first and second markers, and the haplotype of said additional polymorphic marker is determined by restriction fragment length polymorphism (RFLP) analysis or fluorescent depolarization analysis of the amplification product.

3. The method of claim 1, wherein said forward or reverse primers are fluorescently labelled and the remaining primers have divergent 5' extensions, and wherein said amplification product analysis comprises gel electrophoresis and fragment analysis.
